# FASCICULE DE BREVET EUROPEEN

(11) **EP 2 180 869 B1**
(45) Date de publication et mention de la délivrance du brevet: **20.03.2013**
(21) Numéro de dépôt: 08828086.2
(22) Date de dépôt: 15.07.2008
(51) Int. Cl.: A61J 15/00, A61L 27/58

(54) **Sonde de gastrostomie percutanée à collerette interne et à embout biodégradable**
Perkutane Gastrostomiesonde mit Innenring und biologisch abbaubarem Endstück
Percutaneous gastrostomy probe with internal collar and biodegradable end piece

(30) Priorité: 31.07.2007 FR 0705569
(43) Date de publication de la demande: 05.05.2010
(73) Titulaire: Medwin France, 34240 Lamalou les Bains (FR)
(72) Inventeur: RENAUX, Serge, F-34240 Lamalou les Bains (FR); SENESSE, Pieree, 34080 Montpellier (FR)
(74) Mandataire: Gosse, Michel
(86) Numéro de dépôt international: PCT/FR2008/001026
(87) Numéro de publication internationale: WO 2009/027600

(56) Documents cités:
- EP-A- 0 238 952
- WO-A-99/17708
- WO-A-2006/078672
- DE-A1- 19 543 011
- US-A- 4 315 513
- US-A1- 2002 198 440

## Description

### DOMAINE DE L'INVENTION

La présente invention se rapporte aux sondes de gastrostomie percutanée endoscopique (GPE) et radiologique (GPR) permettant l'accès direct à la cavité gastrique pour une alimentation entérale.

### ARRIERE PLAN TECHNOLOGIQUE

La gastrostomie percutanée est actuellement la voie de référence pour l'alimentation entérale prolongée. La simplicité et la rapidité de la technique, l'évolution du matériel font que les gastro-entérologues ou les radiologues sont de plus en plus sollicités et que la pose est accessible à tout endoscopiste ou radiologue.

Il existe deux techniques de pose :
- la technique « Pull » par voie endoscopique, principalement utilisée par les gastro-entérologues : les kits de GPE stériles comprennent généralement un trocart de ponction, un fil métallique double brin, une sonde de gastrostomie tubulaire, un moyen de rétention interne, du type collerette, une collerette de fixation externe : on peut utiliser, pour saisir le fil intragastrique, une pince à corps étrangers type dent de rat ou crocodile, une anse diathermique ou une pince à biopsie ;
- la technique « Push » par la paroi abdominale, principalement utilisée par les radiologues : dans ce cas des ancres permettent d'arrimer l'estomac à la paroi abdominale le temps nécessaire à la formation d'adhérences entre la partie externe de l'estomac et la paroi abdominale, une sonde à ballonnet est alors positionnée à travers la paroi abdominale au moyen d'un dilatateur et d'une canule pelable.

Les sondes sont généralement en silicone ou polyuréthane, matériaux inertes et bien tolérés. Différents calibres ou charrières sont disponibles, les sondes de petit calibre s'obstruant plus facilement.

Il existe des sondes extractibles et non extractibles.

Les sondes non extractibles, pour être remplacées, doivent être sectionnées au ras de l'orifice cutané. Le dispositif interne est ensuite poussé dans l'estomac.

Le moyen de rétention interne peut être soit récupéré par endoscopie, opération qui peut s'avérer délicate, soit évacué par les voies naturelles avec des risques d'occlusion et de perforation intestinale.

L'avantage des sondes non extractibles réside dans leur collerette interne relativement rigide résistant donc bien à une tentative d'arrachement par un patient agité.

Les sondes extractibles ont une collerette interne rétractable, ou un système de rétention dégonflable, permettant leur ablation par l'orifice cutané par traction ferme. L'avantage de ces systèmes souples est de pouvoir franchir une sténose haute, d'éviter une endoscopie mais elles ont moins de résistance à l'arrachement.

Le choix entre sondes extractibles et non extractibles en silicone ou polyuréthane dépend de l'indication, en tenant compte des avantages et inconvénients de chaque type de sonde.

Les sondes extractibles en silicone conviennent pour une nutrition entérale temporaire. Les sondes non extractibles ou en polyuréthane sont plus adaptées pour une nutrition entérale définitive ou chez un patient agité.

Le remplacement de la sonde de gastrostomie peut s'avérer nécessaire en cas d'obstruction, de détérioration du tube (fissuration, porosité, dilatation, colonisation par des candida).

La majorité des dispositifs de remplacement sont des sondes à ballonnet gonflable à l'eau, en silicone. Leur bonne adéquation avec le milieu gastrique et une collerette de rétention externe permet une bonne sécurité d'emploi.

Il existe également le bouton de gastrostomie plus court et à même la peau qui, du fait de son avantage esthétique et de son confort, est indiqué chez le sujet jeune ou ambulatoire. Il ne peut cependant que difficilement être mis en première intention et vient le plus souvent en remplacement d'une sonde déjà positionnée dans l'estomac.

La sonde selon l'invention est du type comprenant :
a) une tubulure destinée à traverser les parois stomacale et abdominale du sujet ;
b) un moyen de rétention interne, solidarisé à ladite tubulure, destiné à être maintenu plaqué contre la face interne de la paroi stomacale et à se désolidariser de ladite tubulure par biodégradation ;
c) une collerette externe, traversée par ladite tubulure, destinée à être plaquée contre la face externe de la paroi abdominale et à exercer, en coopération avec le moyen de rétention interne, une pression apte à plaquer la paroi stomacale contre la paroi abdominale dans la zone de la stomie.

Le document de l'état de la technique le plus proche porte la référence WO99/17708 Le moyen de rétention interne qui y est décrit est constitué :
- d'un ballonnet non séparable de la tubulure provoquant, lors de son passage au travers de la stomie, des traumatismes ;
- d'une collerette biodégradable positionné entre ledit ballonnet et la paroi stomacale et séparable de la tubulure qu'après totale biodégradation.

### RESUME DE L'INVENTION

L'invention vise à réaliser une sonde du genre en question destinée à mettre en oeuvre une solution inédite et originale apte à éliminer les inconvénients susmentionnés.

Elle concerne à cet effet, une sonde de gastrostomie percutanée endoscopique qui se caractérise essentiellement en ce que le moyen de rétention interne est constitué :
a) d'une collerette, plaquée, par l'une de ses faces, contre la paroi stomacale, séparable de la tubulure sous l'effet d'un simple effort de traction, réalisée en un matériau non biodégradable choisi exclusivement pour ses propriétés élastomères facilitant sa mise en place par voie endoscopique et son évacuation par les voies naturelles ;
b) d'un embout, plaqué contre la face opposée de ladite collerette, destiné à la maintenir contre la paroi stomacale, réalisé en un polymère ou copolymère entièrement biodégradable déterminés pour obtenir une liaison avec la tubulure qui possède un seuil de rupture à l'arrachement fonction du temps de formation de la stomie.

Le polymère ou copolymère utilisé est choisi avantageusement parmi des structures PLA et PLA GA.

Les fonctions d'une part de mise en place et d'évacuation par les voies naturelles de la collerette et d'autre part de résistance de celle-ci pendant un temps bien déterminé, ont été séparées permettant ainsi de choisir la collerette pour ses seules propriétés élastomères et l'embout pour ses seules propriétés de biodégradabilité.

Une telle conception a pour avantage essentiel d'obtenir des résultats cliniques les plus appropriés aux fonctions recherchées, à savoir la disparition des risques d'occlusion aux conséquences mortelles et de l'anesthésie nécessitée par l'utilisation d'un endoscope.

Nous sommes donc en présence d'une combinaison de moyens qui n'est pas enseignée dans l'art antérieur cité et qui n'en découle pas de manière évidente pour l'homme de métier.

La période de cicatrisation optimale de la stomie est d'environ 21 jours. Un délai trop court conduirait à une mauvaise cicatrisation de celle-ci avec toutes les conséquences qui pourraient en résulter principalement lors de l'introduction d'une sonde de remplacement.

Les incidents qui en résultent concernent essentiellement l'incarcération de la collerette entre les parois, ou le passage accidentel, dans la cavité abdominale, des produits nutritifs administrés. Ces incidents semblent liés à l'ablation en force de la sonde de gastrostomie au travers de la stomie, conduisant à la désolidarisation de la collerette et provoquant la dilacération de la stomie. Le positionnement de la sonde de remplacement est dans ce cas aléatoire.

### PRESENTATION DES FIGURES

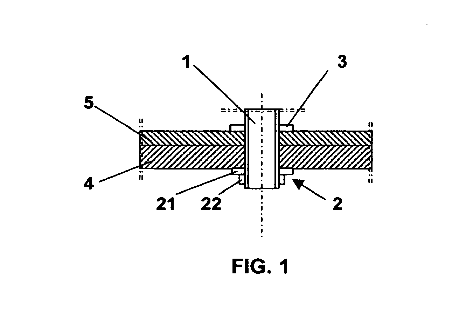

Les caractéristiques et les avantages de l'invention vont apparaître plus clairement à la lecture de la description détaillée qui suit d'au moins un mode de réalisation préféré de celle-ci donné à titre d'exemple non limitatif et représenté au dessin annexé (figure unique) qui représente, en coupe, une vue partielle d'une sonde pourvue d'une collerette et d'un embout prenant en « sandwich » les parois stomacale et abdominale.

### DESCRIPTION DETAILLEE DE L'INVENTION

La sonde de gastrostomie percutanée représentée est du genre comprenant :
a) une tubulure (1) destinée à traverser les parois stomacale (4) et abdominale (5) du sujet ;
b) un moyen de rétention interne (2), solidarisé à ladite tubulure (1), destiné à être maintenu plaqué contre la face interne de la paroi stomacale et à se désolidariser de ladite tubulure par biodégradation ;
c) une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie.

Le moyen de rétention interne est avantageusement constitué :
a) d'une collerette (21), plaquée, par l'une de ses faces, contre la paroi stomacale (4), séparable de la tubulure (1) sous l'effet d'un simple effort de traction, réalisée en un matériau non biodégradable choisi exclusivement pour ses propriétés élastomères facilitant sa mise en place par voie endoscopique et son évacuation par les voies naturelles ;
b) d'un embout (22), plaqué contre la face opposée de ladite collerette (21), destiné à la maintenir contre la paroi stomacale (4), réalisé en un polymère ou copolymère entièrement biodégradable déterminés pour obtenir une liaison avec la tubulure (1) qui possède un seuil de rupture à l'arrachement, par simple traction, fonction du temps de formation de la stomie.

La vitesse de biodégradabilité du moyen de rétention est programmée de sorte que les caractéristiques mécaniques de celui-ci soient maintenues au moins jusqu'à l'adhésion des parois stomacale et abdominale entre elles.

L'embout (22), qui est pourvu d'une ouverture pour le passage de la tubulure (1), peut être solidarisé à cette dernière par collage, soudage, sertissage ou par tout autre moyen connu.

La tubulure (1) est généralement réalisée en un matériau biocompatible non biodégradable comme par exemple du silicone ou du polyuréthane.

Le choix du polymère ou copolymère biodégradable adapté à l'application médicale considérée a fait l'objet de tests consistant à synthétiser divers polymères ou copolymères en des échantillons, immergés dans un modèle de fluide gastrique, ayant des dimensions similaires à celles des moyens de rétention internes biodégradables concernés, capables de se dégrader dans un délai bien déterminé (notamment compris entre 1 et 3 mois), ayant les caractéristiques physiques et mécaniques requises en termes d'élasticité ou de dureté, de changement de forme, de gonflement due à la prise d'eau, de décomposition...

Des tests ont été effectués sur des échantillons réalisés avec les divers polymères ou copolymères des types :
- poly acide lactique comme le PLA 50 (Mn = 21000g/mol) et le PLA 50 (Mn = 46000 g/mol) ;
- poly acide lactique-acide glycolique comme le PLA 37,5 - GA 25 (Mn = 39000 g/mol) ;
- triblocs PLA - PEG (poly éthylène glycol) - PLA comme le PLA 50 - PEG (20000) - PLA 50 (Mn = 277760 g/mol), le PLA 50 - PEG (20000) - PLA 50 (Mn = 100600 g/mol), le PLA 50 - PEG (6000) - PLA 50 (Mn = 56400 g/mol), le PLA 96 - PEG 12000
- PLA 96 (Mn = 68311 g/mol), le PLA 96 - PEG 8000 - PLA 96 (Mn = 71684 g/mol) et les PLA GA - PEG - PLA GA.

C'est dans la catégorie des polymères PLA et plus avantageusement des PLA GA que le choix s'est porté pour réaliser les embouts où les problèmes de mémoire de forme et de souplesse ne se posent pas.

L'embout possède une épaisseur comprise avantageusement entre 1 et 3 mm (non limitatif) pour jouer également sur la vitesse de biodégradabilité.

Des composants ayant pour effet de modifier les caractéristiques mécaniques et la vitesse de dégradation ainsi que la tolérance de l'organisme, peuvent être rajoutés auxdits copolymères

Bien entendu, l'homme de métier sera apte à réaliser l'invention telle que décrite et représentée en appliquant et en adaptant des moyens connus.

Il pourra également prévoir d'autres variantes sans pour cela sortir du cadre de l'invention qui est déterminé par la teneur des revendications.

## Revendications

1. Sonde de gastrostomie percutanée comprenant :
a) une tubulure (1) destinée à traverser les parois stomacale (4) et abdominale (5) du sujet ;
b) un moyen de rétention interne (2), solidarisé à ladite tubulure (1), destiné à être maintenu plaqué contre la face interne de la paroi stomacale et à se désolidariser de ladite tubulure par biodégradation ;
c) une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie ;
**caractérisée en ce que** le moyen de rétention interne (2) est constitué :
a) d'une collerette (21), plaquée, par l'une de ses faces, contre la paroi stomacale (4), séparable de la tubulure (1) sous l'effet d'un simple effort de traction, réalisée en un matériau non biodégradable choisi exclusivement pour ses propriétés élastomères facilitant sa mise en place par voie endoscopique et son évacuation par les voies naturelles ;
b) d'un embout (22), plaqué contre la face opposée de ladite collerette (21), destiné à la maintenir contre la paroi stomacale (4), réalisé en un polymère ou copolymère entièrement biodégradable déterminés pour obtenir une liaison avec la tubulure (1) qui possède un seuil de rupture à l'arrachement fonction du temps de formation de la stomie.

2. Sonde, selon la revendication 1, **caractérisée en ce que** le polymère utilisé est du type PLA.

3. Sonde, selon la revendication 1, **caractérisée en ce que** le copolymère utilisé est du type PLA GA.

4. Sonde, selon la revendication 1, **caractérisée en ce que** l'embout utilisé possède une épaisseur comprise entre 1 et 3 mm.

## Claims

1. Percutaneous gastrostomy catheter comprising:
a) a tubing (1) intended to pass through the subject's stomach (4) and abdominal walls (5);
b) internal retention means (2), rigidly connected to said tubing (1), intended to be kept pressed against the inside of the stomach wall and to be detached from said tubing by biodegradation;
c) an outer collar (3), traversed by the tubing (1), intended to be pressed against the outside of said abdominal wall (5) and to exert, in conjunction with the internal retention means (2), pressure suitable for pressing the stomach wall (4) against the abdominal wall (5) in the stoma region;
**characterised in that** the internal retention means (2) consists of:
a) a collar (21), pressed, by means of one of the faces thereof, against the stomach wall (4), suitable for being separated from the tubing (1) under the effect of a mere pulling force, made of a non-biodegradable material chosen exclusively for the elastomeric properties thereof facilitating the positioning thereof by the endoscopic route and the natural discharge thereof;
b) a tip (22), pressed against the face opposite said collar (21), intended to hold same against the stomach wall (4), made of a predetermined fully biodegradable polymer or copolymer to obtain a connection with the tubing (1) having a tearing threshold that is dependent on the stoma formation time.

2. Catheter, according to claim 1, **characterised in that** the polymer used is PLA type.

3. Catheter, according to claim 1, **characterised in that** the copolymer used is PLA GA type.

4. Catheter, according to claim 1, **characterised in that** the tip used is between 1 and 3 mm thick.

## Patentansprüche

1. Perkutane Gastrostomiesonde, umfassend:
a) einen Schlauch (1), der ausgelegt ist, die Magen- (4) und Bauchwand (5) des Subjekts zu durchqueren;
b) ein internes Rückhaltemittel (2), das fest mit dem Schlauch (1) verbunden ist, ausgelegt, um gegen die innere Seite der Magenwand (4) gedrückt gehalten zu werden und sich durch biologischen Abbau von dem Schlauch zu trennen;
c) einen externen Kragen (3), der von dem Schlauch (1) durchquert wird, ausgelegt, gegen die äußere Seite der Bauchwand (5) gedrückt zu werden und, in Zusammenarbeit mit dem internen Rückhaltmittel (2), einen Druck auszuüben, der dazu geeignet ist, die Magenwand (4) gegen die Bauchwand (5) im Bereich der Ostomie zu drücken;
**dadurch gekennzeichnet, dass** das interne Rückhaltemittel (2) aus Folgendem besteht:
a) einem Kragen (21), der, durch eine seiner Flächen, gegen die Magenwand (4) gedrückt ist, trennbar vom Schlauch (1) unter der Einwirkung einer einfachen Zugkraft, hergestellt aus einem nicht biologisch abbaubaren Material, das ausschließlich aufgrund seiner elastomeren Eigenschaften ausgewählt wurde, die seine Anbringung auf endoskopischem Weg und seine Entfernung auf natürlichen Wegen erleichtern;
b) einem Ansatzstück (22), das gegen die gegenüber liegende Seite des Kragens (21) gedrückt ist, dazu ausgelegt, ihn gegen die Magenwand (4) zu halten, hergestellt aus einem vollständig biologisch abbaubaren Polymer oder Copolymer, dazu ausgelegt, eine Verbindung mit dem Schlauch (1) zu erhalten, die eine Reiß-Bruchschwelle je nach der Zeit der Bildung der Ostomie aufweist.

2. Sonde nach Anspruch 1, **dadurch gekennzeichnet, dass** das verwendete Polymer vom Typ PLA ist.

3. Sonde nach Anspruch 1, **dadurch gekennzeichnet, dass** das verwendete Copolymer vom Typ PLA GA ist.

4. Sonde nach Anspruch 1, **dadurch gekennzeichnet, dass** das Ansatzstück, das verwendet wird, eine Dicke zwischen 1 und 3 mm aufweist.
